# FASCICULE DE BREVET EUROPEEN

(11) **EP 2 459 735 B1**
(45) Date de publication et mention de la délivrance du brevet: **08.10.2014**
(21) Numéro de dépôt: 10752886.1
(22) Date de dépôt: 28.07.2010
(51) Int. Cl.: C12Q 1/04, C12Q 1/37, G01N 33/84

(54) **NOUVEAUX SUBSTRATS DE PEPTIDASE**
NEUARTIGE PEPTIDASE-SUBSTRATE
NOVEL PEPTIDASE SUBSTRATES

(30) Priorité: 30.07.2009 FR 0903755
(43) Date de publication de la demande: 06.06.2012
(73) Titulaire: Biomérieux, 69280 Marcy L'Etoile (FR)
(72) Inventeur: JAMES, Arthur, Cumbria CA13 9 UX (GB); ORENGA, Sylvain, F-01160 Neuville Sur Ain (FR); PERRY, John, Newcastle upon Tyne NE7 7BH (GB); SALWATURA, Vindhya Lakshika, Newcastle Upon Tyne NE4 9JB (GB); STANFORTH, Stephen, Northumberland NE 43 7EH (GB)
(86) Numéro de dépôt international: PCT/FR2010/051599
(87) Numéro de publication internationale: WO 2011/012811

(56) Documents cités:
- WO-A1-2006/030119
- FR-A1- 2 916 763
- ANDERSON ET AL: "Synthesis and evaluation of novel chromogenic peptidase substrates based on 9-(4'-aminophenyl)-10-methylacridinium salts as diagnostic tools in clinical bacteriology", BIOORGANIC & MEDICINAL CHEMISTRY LETTERS, PERGAMON, ELSEVIER SCIENCE, GB, vol. 18, no. 2, 17 novembre 2007 (2007-11-17), pages 832-835, XP022424759, ISSN: 0960-894X
- MANAFI M ET AL: "FLUOROGENIC AND CHROMOGENIC SUBSTRATES USED IN BACTERIAL DIAGNOSTICS", MICROBIOLOGICAL REVIEWS, AMERICAN SOCIETY FOR MICROBIOLOGY, WASHINGTON, DC, US, vol. 55, no. 3, 1 septembre 1991 (1991-09-01), pages 335-348, XP009020939, ISSN: 0146-0749
- DIKOV A ET AL: "NEW FLUOROGENIC SUBSTRATES IN HISTOCHEMISTRY OF PEPTIDASES: I. HISTOCHEMICAL DETERMINATION OF ALANINEAMINOPEPTIDASE", COMPTES RENDUS DE L'ACADEMIE BULGARE DES SCIENCES, SOFIA, GB, vol. 49, no. 4, 1 janvier 1996 (1996-01-01), pages 89-92, XP008056562, ISSN: 0861-1459

## Description

La présente invention concerne de nouveaux composés utilisables à titre d'indicateurs de pH et/ou de substrats enzymatiques pour la détection d'activité peptidase. Ces substrats sont utilisables dans les applications comportant une étape d'hydrolyse enzymatique produisant un signal physico-chimique notamment en microbiologie, biochimie, immunologie, biologie moléculaire, histologie, etc. L'invention concerne également des milieux réactionnels contenant de tels substrats, l'utilisation des substrats ou des milieux pour la détection d'activités peptidases et/ou la différenciation de bactéries à Gram positif par rapport à des bactéries à Gram négatif et des procédés d'utilisation.

Il existe actuellement de très nombreux milieux permettant la détection de microorganismes. Cette détection peut être basée notamment sur l'utilisation de substrats particuliers, spécifiques d'une enzyme du microorganisme que l'on souhaite détecter. D'une manière générale, les substrats synthétiques d'enzymes sont constitués de telle façon que le substrat et le produit de son métabolisme par l'enzyme cible possèdent des propriétés physico-chimiques différentes, permettant de les distinguer et d'évaluer si tout ou partie du substrat a été converti en produit par l'enzyme. Les substrats d'hydrolase, sont généralement constitués d'une première partie spécifique de l'activité enzymatique à révéler, et d'une seconde partie faisant office de marqueur, généralement chromogène ou fluorescent. Ainsi dans le cas des bactéries, par le choix des substrats, selon qu'il y a réaction ou non, il est possible de caractériser la nature d'un microorganisme. Une activité peptidase peut notamment être utilisée pour révéler un groupe, un genre ou une espèce de bactéries. Ainsi, l'activité alanine-aminopeptidase, par exemple, permet de différencier les bactéries à Gram négatif des bactéries à Gram positif.

Des substrats chromogènes enzymatiques pour la détection d'activité peptidase sont connus de l'état de la technique. On peut citer notamment la publication de Manafi (Manafi et al, Microbiol Rev 55(3) : 335-348, 1991), qui est une revue de substrats enzymatiques utilisés en microbiologie. Toutefois, les substrats d'aminopeptidase décrits libèrent par hydrolyse des composés diffusant dans le milieu (beta-naphtylamine, 7-amino-4-methylcoumarine). De ce fait, en milieu réactionnel hétérogène (colonies sur boites de Pétri, coupe histologique...), il n'est pas possible de localiser précisément le lieu de l'hydrolyse. On peut citer également les substrats décrits dans les demandes de brevet WO 98/04735 et WO 99/38995 déposées par la Demanderesse. Toutefois, bien que ces substrats diffusent peu en milieu de culture, ils présentent certains inconvénients : leur synthèse est difficile, la pureté est réduite, les rendements faibles et ils sont toxiques vis-à-vis de certains micro-organismes. D'autres substrats enzymatiques pour la détection d'activité peptidase sont décrits dans la demande de brevet FR 2916763.

La présente invention propose donc l'utilisation de nouveaux composés, soit à titre d'indicateurs de pH, soit à titre de substrats de peptidase, permettant la détection de microorganismes. Comparativement aux substrats existants, ces nouveaux composés sont de synthèse aisée, et peuvent être utilisés notamment en milieux gélifiés pour la détection de micro-organismes car ils produisent une coloration ne diffusant peu ou pas dans le milieu réactionnel. Dans le cadre d'une utilisation comme substrat enzymatique, cela permet de repérer une colonie ou une organelle exprimant une activité peptidase parmi d'autres ne l'exprimant pas.

Avant d'aller plus avant dans la description de l'invention, les définitions ci dessous sont données afin de faciliter l'exposé de l'invention.

Par substrat enzymatique, on entend un substrat pouvant être hydrolysé par une enzyme en un produit permettant la détection, directe ou indirecte d'un microorganisme, d'une cellule ou d'une organelle. Ce substrat comprend notamment une première partie spécifique de l'activité enzymatique à révéler et une seconde partie faisant office de marqueur.

Les composés selon l'invention utilisés comme substrats sont adaptés à une utilisation en cytométrie en flux, car le produit de l'hydrolyse restant principalement localisé dans la cellule exprimant l'activité enzymatique, il est possible de compter spécifiquement les cellules exprimant cette activité, voire de les séparer du reste de l'échantillon.

Les composés selon l'invention utilisés comme substrats sont également bien adaptés à une utilisation en histoenzymologie, car le produit d'hydrolyse restant principalement localisé sur le lieu de l'hydrolyse, il est possible d'identifier spécifiquement les cellules ou organelles exprimant cette activité au sein d'un tissu.

Du fait de leur faible toxicité, les composés selon l'invention sont bien adaptés, respectivement comme indicateurs de pH, ou pour le suivi d'activité peptidase en culture cellulaire.

Les composés selon l'invention sont particulièrement bien adaptés à une utilisation en milieu de détection et/ou d'identification car ils produisent une coloration ou une fluorescence ne diffusant pas dans le milieu réactionnel.

Dans la présente demande, le terme coloration est employé pour couvrir une coloration, absorption de lumière dans le spectre visible, ou une fluorescence, absorption à une longueur d'onde (λₑₓ) et émission à une longueur d'onde supérieure (λₑₘ, λₑₘ > λₑₓ). Les composés de l'invention peuvent être salifiés, c'est à dire sous forme de sel tel que chlorure, bromure, iodure ou trifluoroacétate.

Par indicateur de pH, on entend une substance chimique dont la couleur et/ou la fluorescence varie en fonction des modifications de pH du milieu, lesdites modifications étant liées ou non au métabolisme du ou des microorganismes en croissance sur ledit milieu.

Par peptidase, on entend une enzyme capable de cliver par hydrolyse le groupement amide formé entre le reste acyle d'un peptide et une amine primaire. Par aminopeptidase, on entend une enzyme capable de cliver par hydrolyse le groupement amide formé entre un acyle d'acide aminé et une amine primaire. Dans la présente demande, le terme « peptidase » peut désigner, selon les cas, tant une peptidase qu'une aminopeptidase telles que définies précédemment.

Par peptide, on entend une chaîne peptidique comprenant de 1 à 10 acides aminés, préférentiellement de 1 à 4 acides aminés. Préférentiellement, le peptide est un di-Alanine ou tri-Alanine. Par acide aminé, on entend tout acide aminé connu de l'homme du métier, naturel ou non. Selon un mode particulier de réalisation de l'invention, l'acide aminé est une beta-Alanine ou L-Alanine ou D-Alanine, ou une Glycine, Pyrroglutamyl, ...

Ledit peptide peut comprendre un agent de blocage en son extrémité N terminale. Les agents de blocage selon l'invention comprennent tout agent de blocage connu de l'homme du métier qui est capable de protéger les amines. A titre d'exemple, on peut citer le t-Butoxycarbonyle (N-tBOC), le 9-Fluorenyloxycarbonyle, un agent de solubilisation tel que le Succinyle, ou bien un acide aminé non métabolisable, c'est-à-dire non naturel, tel que l'acide pipécolique ou la forme D d'un acide aminé, tel que la D-Phénylalanine. Les agents de blocage ne sont pas systématiquement présents dans les composés de l'invention.

Par groupement alkyle, on entend une chaîne de groupements hydrocarbonés saturés, tel que notamment un alkyle en C₁-C₆, c'est a dire un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Par groupement aryle, on entend un groupement fonctionnel (ou substituant) qui dérive d'un noyau aromatique tel que notamment un noyau aromatique en C₆-C₁₀, notamment phényle, benzyle, 1-naphtyle ou 2-naphtyle.

Par groupement carboxyle, on entend notamment un groupe fonctionnel composé d'un atome de carbone, lié par une double liaison à un premier atome d'oxygène, et par une liaison simple à un second atome d'oxygène, lui-même chargé négativement ou relié à un atome d'hydrogène. En fonction du pKₐ de la molécule et du pH du milieu, le groupement carboxyle peut être sous forme ionisée, c'est à dire sans H lié au second atome d'oxygène, qui est alors chargé négativement.

Par milieu réactionnel, on entend un milieu comprenant tous les éléments nécessaires à l'expression d'un métabolisme et/ou à la croissance de microorganismes, d'une cellule ou d'une organelle. Ce milieu réactionnel peut être utilisé en cytométrie en flux, histoenzymologie, culture cellulaire ... ou en tant que milieu de détection et/ou d'identification de microorganismes.

Le milieu réactionnel peut comprendre un ou plusieurs éléments en combinaison, tels que des acides aminés, des peptones, des hydrates de carbone, des nucléotides, des minéraux, des vitamines, des antibiotiques, des ténsioactifs, des tampons, des sels de phosphate, d'ammonium, de sodium, de métaux.

Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant ...

Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine ou de l'agarose. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu réactionnel peut être un milieu de détection et/ou d'identification, c'est à dire un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture.

Par échantillon biologique, on entend un échantillon clinique, issu d'un prélèvement de liquide biologique ou un échantillon alimentaire, issu de tout type d'aliment ou un échantillon cosmétique ou pharmaceutique issu de toute préparation cosmétique ou pharmaceutique. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'oeufs, de légumes, de mayonnaise, de fromage ; de poisson..., un échantillon alimentaire issu d'une alimentation destinée aux animaux, tel que notamment un échantillon issu de farines animales. L'échantillon peut également être issu d'un prélèvement de l'environnement clinique, d'élevage ou de production alimentaire, cosmétique ou pharmaceutique. Par prélèvement d'environnement, on entend notamment un prélèvement de surface, de liquide, de matière première ou de produit. Par échantillon, on entend donc le prélèvement en lui même (écouvillon, selles, aliments, ...) aussi bien que des colonies de microorganismes issus dudit prélèvement (par exemple après isolement sur un milieu de culture gélifié) ou un milieu contenant des microorganismes issus dudit prélèvement (par exemple un bouillon d'enrichissement ensemencé avec ledit prélèvement)

Au sens de la présente invention, le terme microorganisme recouvre les bactéries, les levures, les moisissures et plus généralement, les organismes généralement unicellulaires, invisibles à l'oeil nu, qui peuvent être multipliés et manipulés en laboratoire.

A titre de bactéries à Gram négatif, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Citrobacter, Hafnia, Edwardsiella, Aeromonas, Moraxella, Pasteurella, Providencia, Actinobacillus, Alcaligenes, Bordetella, Cedecea, Erwinia, Pantoea, Ralstonia, Stenotrophomonas, Xanthomonas* et *Legionella.* A titre de bactéries à Gram positif, on peut citer les bactéries des genres suivants : *Aerococcus, Enterococcus, Streptococcus, Staphylococcus, Bacillus, Lactobacillus, Listeria, Clostridium, Gardnerella, Kocuria, Lactococcus, Leuconostoc, Micrococcus, Falkamia, Gemella, Pediococcus, Mycobacterium* et *Corynebacterium.*
A titre de levures, on peut citer les levures des genres suivants : *Candida, Cryptococcus, Saccharomyces* et *Trichosporon.* Préférentiellement, le microorganismes est choisi parmi *Escherichia coli, Serratia marcescens, Enterobacter cloacae, Enterobacter aerogenes, Citrobacter freundii, Klebsiella pneumoniae, Pseudomonas aeruginosa, Enterococcus faecalis.*

A ce titre l'invention concerne l'utilisation d'un composé de la formule (I) suivante, comme substrat enzymatique pour la détection d'une activité peptidase et/ou une variation de pH : 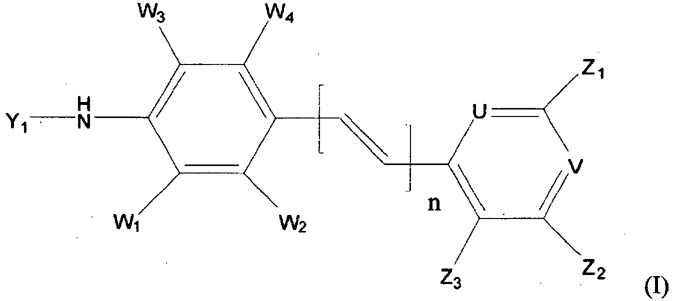 selon laquelle :
- Y₁ est un peptide, H ou un alkyl
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
- n = 0, 1 ou 2
- U et V sont N, N⁺R, CZ₄, R étant H, alkyle, aralkyle, aryle, alcanoïque ou alkylsulfonique. Préférentiellement, si U est CZ₄, V est N ou N⁺R ; si V est CZ₄, U est N ou N⁺R.
- Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels

Lorsque ledit composé de formule (I) est utilisé pour détecter uniquement une variation de pH, Y₁ est H ou un alkyl.

Lorsque ledit composé de formule (I) est utilisé pour la détection d'une activité peptidase, Y₁ est un peptide.

Lorsque ledit composé de formule (I) est utilisé pour la détection d'une activité peptidase et d'une variation de pH, Y₁ est un peptide.

Selon un mode préféré de réalisation de l'invention, Y₁ est un peptide, préférentiellement choisi la glycine, l'alanine, préférentiellement l'alanine Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CZ4, préférentiellement CH Selon un mode préféré de réalisation de l'invention, V est N⁺R, préférentiellement N⁺CH3

Selon un mode préféré de réalisation de l'invention, Z₁, Z₂, Z₃ et Z₄ sont H

Selon un mode préféré de réalisation de l'invention, ledit composé est choisi parmi Glycyl-4-(4'-amidostyryl)-N-methyl-pyridinium chloride, L-Alanyl-4-(4'-amidostyryl)-N-methyl-pyridinium TFA, ß-alanyl-4-(4'-amidostyryl)-N-methyl-pyridinium chloride, L-Alanyl-4-(4'-amidostyryl)-pyridine perchlorate (unquaternised)

Selon un mode préféré de réalisation de l'invention,
- ledit peptide est l'alanine
- n = 1
- W₁, W₂, W₃ et W₄ sont indépendamment H
- U est CZ4, préférentiellement CH
- V est N⁺R, préférentiellement N⁺CH3
- Z₁, Z₂, Z₃ et Z₄ sont H

L'invention concerne également un procédé pour la détection chez des micro-organismes d'une activité peptidase et/ou d'une variation de pH, caractérisé en ce qu'il comprend, ou est constitué par les étapes suivantes :
a) disposer d'un milieu de détection et/ou d'identification comprenant d'un composé de la formule (I) suivante : 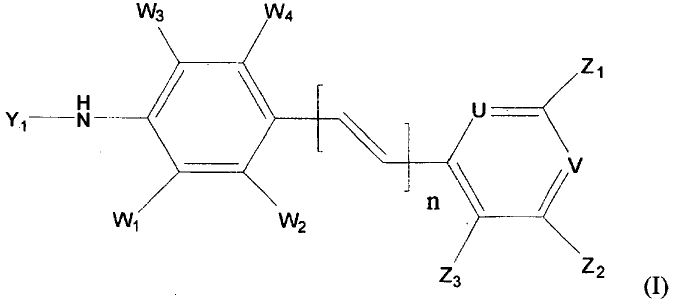 selon laquelle :
   - Y₁ est un peptide, H ou un alkyl
   - W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
   - n = 0, 1 ou 2
   - U et V sont N, N⁺R, CZ₄, R étant H, alkyle, aralkyle, aryle, alcanoïque ou alkylsulfonique. Préférentiellement, si U est CZ₄, V est N ou N⁺R ; si V est CZ₄, U est N ou N⁺R.
   - Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels
b) ensemencer le milieu avec un échantillon biologique à tester,
c) laisser incuber, et
d) révéler la présence d'au moins une activité peptidase ou une variation de pH

L'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase ou la variation de pH, seule ou en combinaison avec au moins une autre activité enzymatique.

Lorsque ledit procédé est un procédé pour détecter uniquement une variation de pH, Y₁ est H ou un alkyl

Lorsque ledit procédé est un procédé pour la détection chez des micro-organismes d'une activité peptidase, Y₁ est un peptide.

Lorsque ledit procédé est un procédé pour la détection chez des micro-organismes d'une activité peptidase et d'une variation de pH, Y₁ est un peptide.

Selon un mode préféré de réalisation de l'invention, Y₁ est un peptide, préférentiellement choisi parmi la glycine, l'alanine, préférentiellement l'alanine Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CZ4, préférentiellement CH Selon un mode préféré de réalisation de l'invention, V est N⁺R, préférentiellement N⁺CH3

Selon un mode préféré de réalisation de l'invention, Z₁, Z₂, Z₃ et Z₄ sont H

Selon un mode préféré de réalisation de l'invention, ledit composé est choisi parmi Glycyl-4-(4'-amidostyryl)-N-methyl-pyridinium chloride, L-Alanyl-4-(4'-amidostyryl)-N-methyl-pyridinium TFA, ß-alanyl-4-(4'-amidostyryl)-N-methyl-pyridinium chloride, L-Alanyl-4-(4'-amidostyryl)-pyridine perchlorate (unquaternised)

Selon un mode préféré de réalisation de l'invention,
- ledit peptide est l'alanine
- n = 1
- W₁, W₂, W₃ et W₄ sont indépendamment H
- U est CZ4, préférentiellement CH
- V est N⁺R, préférentiellement N⁺CH3
- Z₁, Z₂, Z₃ et Z₄ sont H

Préférentiellement, le microorganismes est choisi parmi *Escherichia coli, Serratia marcescens, Enterobacter cloacae, Enterobacter aerogenes, Citrobacter freundii, Klebsiella pneumoniae, Pseudomonas aeruginosa, Enterococcus faecalis.*

La description concerne également un procédé pour la différenciation chez des bactéries de leur appartenance aux bactéries à Gram positif ou aux bactéries à Gram négatif, caractérisé en ce qu'il comprend ou est constitué par les étapes suivantes :
a) disposer d'un milieu de détection et/ou d'identification comprenant d'un composé de la formule (I) suivante, comme substrat enzymatique pour la détection d'une activité peptidase et/ou d'une variation de pH : 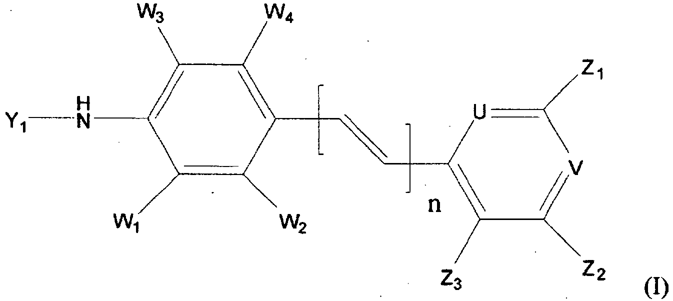 selon laquelle :
   - Y₁ est un peptide, H ou un alkyl
   - W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
   - n = 0, 1 ou 2
   - U et V sont N, N⁺R, CZ₄, R étant H, alkyle, aralkyle, aryle, alcanoïque ou alkylsulfonique. Préférentiellement, si U est CZ₄, V est N ou N⁺R ; si V est CZ₄, U est N ou N⁺R.
   - Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
   et leurs sels.
b) ensemencer le milieu avec un échantillon biologique à tester,
c) laisser incuber, et
d) révéler la présence d'au moins une activité peptidase

Comme indiqué précédemment, l'ensemencement des microorganismes peut être réalisé par toutes les techniques d'ensemencement connues de l'homme du métier. Une étape d'incubation peut être réalisée à une température pour laquelle l'activité enzymatique que l'on souhaite détecter est optimale, que l'homme du métier peut choisir aisément selon l'activité enzymatique à détecter. L'étape d) peut s'effectuer par un examen visuel, par colorimétrie ou fluorimétrie. Lors de l'étape d), on peut révéler la présence de l'activité peptidase, seule ou en combinaison avec d'autres activités enzymatiques. Dans certains cas, il peut être avantageux d'effectuer l'étape d) en présence d'un acide, tel que l'acide acétique.

Lorsque ledit procédé est un procédé pour détecter uniquement une variation de pH, Y₁ est H ou un alkyl

Lorsque ledit procédé est un procédé pour la détection chez des micro-organismes d'une activité peptidase, Y₁ est un peptide.

Lorsque ledit procédé est un procédé pour la détection chez des micro-organismes d'une activité peptidase et d'une variation de pH, Y₁ est un peptide.

Selon un mode préféré de réalisation, Y₁ est un peptide, préférentiellement choisi parmi la glycine, l'alanine, préférentiellement l'alanine Selon un mode préféré de réalisation, n = 1

Selon un mode préféré de réalisation, W₁, W₂, W₃ et W₄ sont indépendamment H

Selon un mode préféré de réalisation, U est CZ4, préférentiellement CH Selon un mode préféré de réalisation, V est N⁺R, préférentiellement N⁺CH3

Selon un mode préféré de réalisation, Z₁, Z₂, Z₃ et Z₄ sont H

Selon un mode préféré de réalisation, ledit composé est choisi parmi Glycyl-4-(4'-amidostyryl)-N-methyl-pyridinium chloride, L-Alanyl-4-(4'-amidostyryl)-N-methyl-pyridinium TFA, ß-alanyl-4-(4'-amidostyryl)-N-methyl-pyridinium chloride, L-Alanyl-4-(4'-amidostyryl)-pyridine perchlorate (unquaternised)

Selon un mode préféré de réalisation de l'invention,
- ledit peptide est l'alanine
- n = 1
- W₁, W₂, W₃ et W₄ sont indépendamment H
- U est CZ4, préférentiellement CH
- V est N⁺R, préférentiellement N⁺CH3
- Z₁, Z₂, Z₃ et Z₄ sont H

Préférentiellement, le microorganismes est choisi parmi *Escherichia coli, Serratia marcescens, Enterobacter cloacae, Enterobacter aerogenes, Citrobacter freundü, Klebsiella pneumoniae, Pseudomonas aeruginosa, Enterococcus faecalis.*

L'invention concerne également un milieu de détection et/ou d'identification de microorganismes comprenant un composé de la formule (I) suivante pour détecter une activité peptidase et/ou une variation de pH : 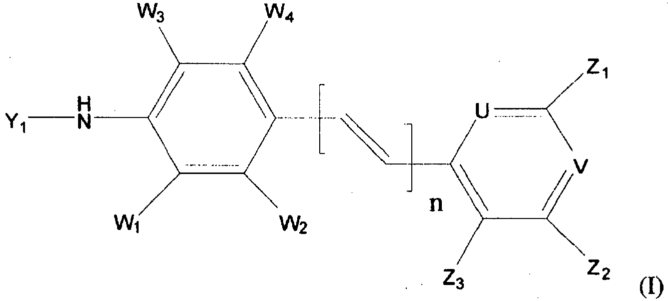 selon laquelle :
- Y₁ est un peptide, H ou un alkyl
- W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
- n = 0, 1 ou 2
- U et V sont N, N⁺R, CZ₄, R étant H, alkyle, aralkyle, aryle, alcanoïque ou alkylsulfonique. Préférentiellement, si U est CZ₄, V est N ou N⁺R ; si V est CZ₄, U est N ou N⁺R.
- Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels

Lorsque ledit milieu est un milieu pour détecter uniquement une seule variation de pH, Y₁ est H ou un alkyl.

Lorsque ledit milieu est un milieu pour détecter une activité peptidase, Y₁ est un peptide.

Lorsque ledit milieu est un milieu pour détecter une activité peptidase, et une variation de pH, Y₁ est un peptide.

Selon un mode préféré de réalisation de l'invention, Y₁ est un peptide, préférentiellement choisi parmi la glycine, l'alanine, préférentiellement l'alanine.

Selon un mode préféré de réalisation de l'invention, n = 1

Selon un mode préféré de réalisation de l'invention, W₁, W₂, W₃ et W₄ sont indépendamment H

Selon un mode préféré de réalisation de l'invention, U est CZ4, préférentiellement CH Selon un mode préféré de réalisation de l'invention, V est N⁺R, préférentiellement N⁺CH3

Selon un mode préféré de réalisation de l'invention, Z₁, Z₂, Z₃ et Z₄ sont H

Selon un mode préféré de réalisation de l'invention, ledit composé est choisi parmi Glycyl-4-(4'-amidostyryl)-N-methyl-pyridinium chloride, L-Alanyl-4-(4'-amidostyryl)-N-methyl-pyridinium TFA, ß-alanyl-4-(4'-amidostyryl)-N-methyl-pyridinium chloride, L-Alanyl-4-(4'-amidostyryl)-pyridine perchlorate (unquaternised)

Selon un mode préféré de réalisation de l'invention,
- ledit peptide est l'alanine
- n = 1
- W₁, W₂, W₃ et W₄ sont indépendamment H
- U est CZ4, préférentiellement CH
- V est N⁺R, préférentiellement N⁺CH3
- Z₁, Z₂, Z₃ et Z₄ sont H

Préférentiellement, le microorganismes est choisi parmi *Escherichia coli, Serratia marcescens, Enterobacter cloacae, Enterobacter aerogenes, Citrobacter freundii Klebsiella pneumoniae, Pseudomonas aeruginosa, Enterococcus faecalis.*

Préférentiellement, ledit milieu réactionnel est un milieu de détection et/ou d'identification de microorganismes, ledit milieu comprenant au moins une molécule utilisée à titre de substrat enzymatique ou d'indicateur de pH telle que définie ci avant. Préférentiellement, ledit composé, substrat enzymatique ou indicateur de pH, est à une concentration comprise entre 1 et 1000 mg/l, préférentiellement entre 10 et 500 mg/l. Selon un mode particulier de réalisation de l'invention, ledit milieu de détection et/ou d'identification selon l'invention comprend en outre au moins un autre substrat enzymatique, spécifique d'une activité enzymatique différente de l'activité peptidase détectée par la molécule selon l'invention.

Selon un autre mode particulier de réalisation de l'invention, ledit milieu de détection et/ou d'identification selon l'invention comprend en outre au moins un substrat spécifique d'une activité enzymatique différente de celle mise en évidence par la variation de pH.

Le métabolisme enzymatique du ou des autres substrats génère un signal détectable, différent du signal généré par le composé selon l'invention utilisé à titre de substrat enzymatique ou d'indicateur de pH, comme par exemple des produits colorés ou fluorescents différents, pour permettre la mise en évidence comme la détection et/ou l'identification et/ou la quantification d'un ou plusieurs microorganismes. A titre d'autre substrat spécifique, on peut utiliser tout autre substrat classiquement utilisé dans la détection des microorganismes. La concentration de l'autre substrat enzymatique spécifique est généralement comprise entre 0,01 et 1 g/l. L'homme du métier pourra déterminer facilement une telle concentration en fonction du substrat utilisé. A titre indicatif, il est possible de combiner les composés selon l'invention avec des substrats enzymatiques de peptidase, d'osidase, d'estérase ou de réductase. En particulier, il est possible d'associer un substrat selon l'invention pour lequel le peptide est une ß-Alanine avec un substrat d'osidase, tel que le 5-Bromo-4-chloro-3-indolyl-ß-glucoside, ou l'Alizarine-ßgalactoside. Il est également possible d'associer un substrat selon l'invention pour lequel le peptide est la L-Alanine avec un substrat d'estérase, tel que le 5-Bromo-6-chloro-3-indoxyl-octanoate ou le 5-Bromo-3-indoxyl-phosphate.

Selon un mode particulier de réalisation de l'invention, ledit milieu de détection et/ou d'identification selon l'invention comprend en outre au moins un autre substrat enzymatique spécifique de l'activité peptidase ou spécifique de l'activité enzymatique détectée par un composé selon l'invention utilisé à titre d'indicateur de pH. Par le choix particulier de substrats, il est alors possible d'identifier des groupes de microorganismes exprimant une même activité enzymatique. La concentration de l'autre substrat enzymatique spécifique est généralement comprise entre 0,01 et 1 g/l. L'homme du métier pourra déterminer facilement une telle concentration en fonction du substrat utilisé. En particulier, il est possible d'associer un substrat selon l'invention pour lequel le peptide est une L-Alanine avec un substrat de L-Alanine aminopeptidase décrit dans la demande WO2006030119, tel que la L-Alanine-pentyl-résorufamine. Selon un mode particulier de réalisation de l'invention, ledit milieu de détection et/ou d'identification selon l'invention comprend en outre au moins un indicateur métabolique, spécifique d'une activité métabolique différente de celle détectée par le composé selon l'invention utilisé à titre de substrat ou d'indicateur de pH.

Cet indicateur métabolique peut notamment être une source de Carbone ou d'Azote associée ou non à un réactif révélant son métabolisme. Selon un mode particulier, la source de Carbone ou d'Azote est associée à un indicateur de pH différent du composé selon l'invention utilisé à ce titre. Selon un autre mode particulier, la source de Carbone ou d'Azote est associée à un cation. Selon un autre mode particulier l'indicateur métabolique permet de détecter une activité tryptophanase et associe du Tryptophane et un réactif permettant de détecter la production d'Indole.

Les exemples ci après sont donnés à titre illustratif.

### Exemple 1- Synthèse de substrats

La 4-(4'-Aminostyryl)-pyridine était préparée suivant la méthode de Royer (R. Royer, Journal of the Chemical Society, 1947, 560-561).

L'aminoacylation de ces composés afin d'obtenir du 4-(4'-aminostyryl)-aryle avec un acide aminé protégé était effectuée suivant la méthode des anhydrides mixés.

Autre exemple de substrat typique obtenu d'une façon similaire à celle mentionné ci-dessus:
Glycine-N-méthyl-4-(4'-aminostyryl)-pyridinium dichlorée.
¹H-NMR: (DMSO) δ 3.83 (2H, s, >C*H*₂), 4.25 (3H, s, NC*H*₃), 7.43 (1H, d, J=16 Hz, =C*H*-), 7.78 (4H, s, Ar-*H*), 7.99 (1H, d, J=16 Hz, =C*H*-), 8.18 (2H, d, J=5 Hz, Py-*H*), 8.28 (1H, broad s, >N*H*), 8.87 (2H, d, J=5 Hz, Py-*H*).

### Exemple 2 - Utilisation de substrats de formule I selon l'invention pour détecter une variation de pH

### a) Indicateur de pH

4,4' Aminostyryl-pyridine a été synthétisé comme décrit à l'exemple 1

### b) Préparation du milieu

| Composition | en g/l |
|---|---|
| Peptones | 6,25 |
| NaCl | 5 |
| Lactose | 10 |
| Saccharose | 10 |
| 4,4' Aminostyryl-pyridine | 0,05 |
| 5-Bromo-4-chloro-3-indoxyl-ß-D-glucopyranoside | 0,05 |
| Agar | 13 |

Le milieu ci-après est autoclavé puis réparti en boîte de Petri de 90mm de diamètre à raison de 20ml par boîte.

### c) Ensemencement et incubation

Sept souches de micro-organismes issues de la collection NCTC et appartenant à différentes espèces de bactéries sont ensemencées sur ces milieux par isolement en quadrant de 10µl d'une suspension à 0,5 McFarland diluée au 20^{e}. Les milieux sont incubés 24 heures à 37°C, puis les colonies formées sont examinées visuellement.

### d) Lecture des résultats

Les résultats obtenus sont présentés dans le tableau 1.

**Tableau 1: Couleur des colonies en présence de l'indicateur de pH selon l'invention**

| **Espèce** | **Numéro NCTC** | **Couleur des colonies** |
|---|---|---|
| *Citrobacter freundii* | 9750 | Jaune |
| *Klebsiella pneumoniae* | 10 896 | Verte |
| *Enterobacter cloacae* | 11 936 | Verte |
| *Enterobacter aerogenes* | 10 102 | Verte |
| *Escherichia coli* | 12076 | Jaune |
| *Morganella morganii* | 235 | Incolore |
| *Shigella boydii* | 9 327 | Incolore |

Le milieu selon l'invention permet de distinguer potentiellement 4 groupes (3 parmi les souches testées dans cette expérience) de microorganismes selon qu'ils acidifient ou non le milieu par métabolisme du Lactose et/ou du Saccharose : virage au jaune de l'indicateur de pH et qu'ils hydrolysent ou non le 5-Bromo-4-chloro-3-indoxyl-ß-D-glucopyranoside : coloration bleu des colonies. Les colonies appartenant aux espèces *Klebsiella pneumoniae, Enterobacter cloacae, Enterobacter aerogenes* qui expriment les 2 activités apparaissent vertes (mélange de jaune et de bleu), celles d'*Escherichia coli* et *Citrobacter freundii* qui acidifient le milieu par métabolisme du Lactose et/ou du Saccharose apparaissent jaunes et celles de *Morganella morganii* et *Shigella boydii* qui n'expriment aucune des deux activités apparaissent incolores.

### Exemple 3 - Utilisation de substrats de formule I selon l'invention pour détecter une activité peptidase

### a) Substrat de peptidase

### Le ß-Alanyl-4-(4'-amidostyryl)-N-methyl-pyridinium chloride (β-Ala-ASPM⁺) a été synthétisé comme décrit à l'exemple 1

### b) Préparation du milieu

40mg de chacun des substrats ont été dissouts dans 4ml de Diméthylsulfoxide (80mg pour le Asn-ASQM⁺) et ajouté à 400ml de milieu Columbia préalablement autoclavé. Les 6 milieux ont été répartis en boîte de Petri de 90mm de diamètre à raison de 20ml par boîte.

### c) Ensemencement et incubation

Dix-sept souches de micro-organismes issues de collections et appartenant à différentes espèces de bactéries et levures sont ensemencées en spot d'environ 10 000 unités formant colonies.

Les milieux sont incubés 24 heures à 37°C, puis les colonies formées sont examinées visuellement, avec ou sans ajout d'acide, et sous lampe UV à 365nm.

### d) Lecture des résultats

Les résultats obtenus sont présentés dans le tableau 2.

**Tableau 2 : Couleur des colonies en présence de substrats de peptidase selon l'invention**

| Espèce | β-Ala-ASPM⁺ | | |
|---|---|---|---|
| Numéro de collection | Crois. | Coul. | Fluo. |
| *Escherichia coli* NCTC 10 418 | 2 | Vert | Jaune Pâle |
| *Serratia marcescens* NCTC 10 211 | 2 | Orange | Jaune Pâle |
| *Pseudomonas aeruginosa* NCTC 10 662 | 2 | Orange | Jaune Pâle |
| *Yersinia enterocolitica* NCTC 11 176 | 2 | Vert pâle | |
| *Salmonella typhimurium* NCTC 74 | 1 | Vert pâle | |
| *Enterobacter cloacae* NCTC 11 936 | 2 | Vert | |
| *Providencia rettgeri* NCTC 7 475 | 2 | Vert | |
| *Bacillus subtilis* NCTC 9 372 | 2 | Vert pâle | |
| *Enterococcus faecalis* NCTC 775 | 2 | Vert pâle | |
| *Enterococcus faecium* NCTC 7 171 | 2 | Vert pâle | |
| *Staphylococcus epidermidis* NCTC 11 047 | 2 | Vert | |
| *Staphylococcus aureus* NCTC 6 571 | 2 | Vert | |
| *Staphylococcus aureus* NCTC 11 939 | 2 | Vert | |
| *Streptococcus pyogenes* NCTC 8 306 | 1 | Vert pâle | |
| *Listeria monocytogenes* NCTC 11 994 | 2 | Vert pâle | |
| *Candida albicans* ATCC 90 028 | 2 | Vert pâle | |
| *Candida glabrata* NCPF 3 943 | 1 | Vert pâle | |

| | | | |
|---|---|---|---|
| Crois : croissance, Fluo. : fluorescence des colonies, Coul. : couleur des colonies PdC : pas de croissance, 0,5 : croissance faible, 1 : croissance modérée, 2 : bonne croissance, - : pas de couleur / fluorescence | | | |

### e) Conclusion

Sur les milieux selon l'invention, il est possible de détecter des activités peptidase de micro-organismes grâce à la fluorescence ou à la coloration des colonies.

Le substrat selon l'invention permet la croissance de tout type de microorganismes : bactéries à Gram négatif, bactéries à Gram positif, levures, ...

Par les différentes variations de structure des substrats de peptidase selon l'invention, il est possible de distinguer différents groupes de microorganismes. De plus, la coloration ne diffusant pas dans le milieu réactionnel, il est possible de distinguer et de compter les cellules ou colonies exprimant l'activité peptidase de celles ne l'exprimant pas.

## Revendications

1. Utilisation d'un composé de la formule (I) suivante, comme substrat enzymatique pour la détection d'une activité peptidase et/ou une variation de pH : 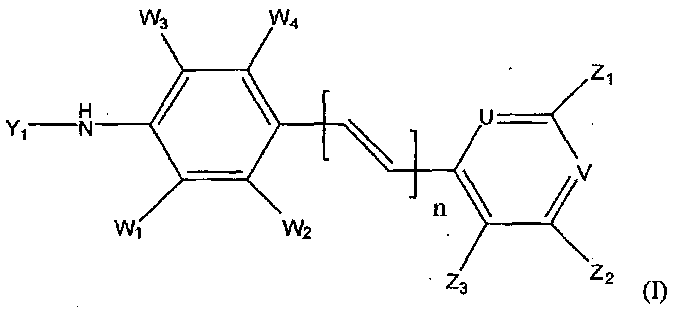 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
• n = 0, 1 ou 2
• U et V sont N, N⁺R, CZ₄, R étant H, alkyle, aralkyle, aryle, alcanoïque ou alkylsulfonique
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels

2. Utilisation d'un composé de formule I selon la revendication 1 selon laquelle Y₁ est un peptide, preferentiellement choisi parmi est l'alanine, la glycine

3. Utilisation d'un composé de formule I selon la revendication 1 ou 2 selon laquelle n = 1

4. Utilisation d'un composé de formule I selon l'une quelconque des revendications 1 à 3 selon laquelle W₁, W₂, W₃ et W₄ sont indépendamment H

5. Utilisation d'un composé de formule I selon l'une quelconque des revendications 1 à 4 selon laquelle U est CZ₄, préférentiellement CH

6. Utilisation d'un composé de formule I selon l'une quelconque des revendications 1 à 5 selon laquelle V est N⁺R, préférentiellement N⁺CH3

7. Utilisation d'un composé de formule I selon l'une quelconque des revendications 1 à 6 selon laquelle Z₁, Z₂, Z₃ et Z₄ sont H

8. Utilisation d'un composé de formule I selon la revendication 1 selon laquelle ledit composé est choisi parmi Glycyl-4-(4'-amidostyryl)-N-methyl-pyridinium chloride, L-Alanyl-4-(4'-amidostyryl)-N-methyl-pyridinium TFA, ß-alanyl-4-(4'-amidostyryl)-N-methyl-pyridinium chloride, L-Alanyl-4-(4'-amidostyryl)-pyridine perchlorate (unquaternised)

9. Procédé pour la détection chez des micro-organismes d'une activité peptidase et/ou d'une variation de pH, **caractérisé en ce qu'**il comprend les étapes suivantes:
a) disposer d'un milieu de détection et/ou d'identification comprenant d'un composé de la formule (I) suivante : 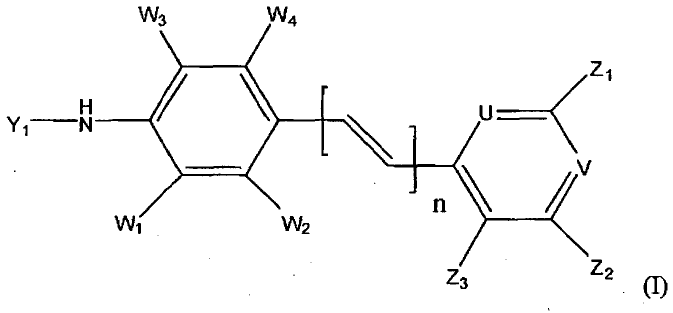 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
• n = 0, 1 ou 2
• U et V sont N, N⁺R, CZ₄, R étant H, alkyle, aralkyle, aryle, alcanoïque ou alkylsulfonique
• Z₁, Z₂, Z₃ et 2₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels
b) ensemencer le milieu avec un échantillon biologique à tester,
c) laisser incuber, et
d) révéler la présence d'au moins une activité peptidase ou une variation de pH

10. Milieu de détection et/ou d'identification de microorganismes comprenant un composé de la formule (I) suivante pour détecter une activité peptidase et/ou une variation de pH : 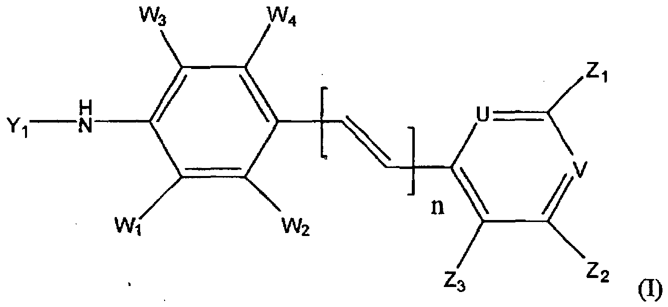 selon laquelle :
• Y₁ est un peptide, H ou un alkyl
• W₁, W₂, W₃ et W₄ sont indépendamment H, Br, Cl, F, I, alkyle, alkoxy, thiométhyle, perfluoroalkyle, nitro, cyano, carboxyle (incluant ses esters ou amides) ou toute combinaison de ceux-ci.
• n = 0, 1 ou 2
• U et V sont N, N⁺R, CZ₄, R étant H, alkyle, aralkyle, aryle, alcanoïque ou alkylsulfonique.
• Z₁, Z₂, Z₃ et Z₄ étant indépendamment H, Br, Cl, F, I, alkyle, aryle, alkoxy, perfluoroalkyle, nitro, cyano, carboxyle, sulfonyle, incluant les esters ou amides de carboxyle ou sulfonyle
et leurs sels.

## Patentansprüche

1. Verwendung einer Verbindung der folgenden Formel (I) als Enzymsubstrat zum Nachweis einer Peptidase-Aktivität und/oder einer Veränderung des pH-Werts: 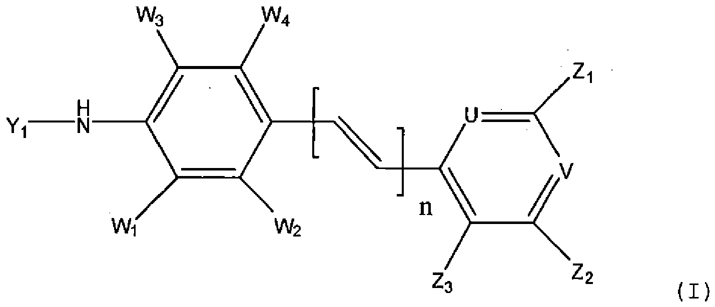 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U und V für N, N⁺R oder CZ₄ stehen, R für H, Alkyl, Aralkyl, Aryl, Alkanoyl oder Alkylsulfonyl steht
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

2. Verwendung einer Verbindung der Formel I nach Anspruch 1, wobei Y₁ ein Peptid ist, das vorzugsweise aus Alanin oder Glycin ausgewählt wird.

3. Verwendung einer Verbindung der Formel I nach Anspruch 1 oder 2, wobei n = 1.

4. Verwendung einer Verbindung der Formel I nach einem der Ansprüche 1 bis 3, wobei W₁, W₂, W₃ und W₄ unabhängig für H stehen.

5. Verwendung einer Verbindung der Formel I nach einem der Ansprüche 1 bis 4, wobei U für CZ₄, vorzugsweise CH, steht.

6. Verwendung einer Verbindung der Formel I nach einem der Ansprüche 1 bis 5, wobei V für N⁺R, vorzugsweise N⁺CH₃, steht.

7. Verwendung einer Verbindung der Formel I nach einem der Ansprüche 1 bis 6, wobei Z₁, Z₂, Z₃ und Z₄ für H stehen.

8. Verwendung einer Verbindung der Formel I nach Anspruch 1, wobei diese Verbindung aus Folgenden ausgewählt wird: glycyl-4-(4'-amidostyryl)-N-methylpyridinium chloride, L-alanyl-4-(4'-amidostyryl)-N-methylpyridinium TFA, β-alanyl-4-(4'-amidostyryl)-N-methylpyridinium chloride and L-alanyl-4-(4'-amidostyryl)pyridine perchlorate (nicht quaternierten).

9. Verfahren zum Nachweis einer Peptidase-Aktivität und/oder einer Veränderung des pH-Werts in Mikroorganismen, **dadurch gekennzeichnet, dass** es die folgenden Schritte umfasst:
a) Bereitstellen eines Nachweis- und/oder Identifikationsmediums, umfassend eine Verbindung der folgenden Formel I: 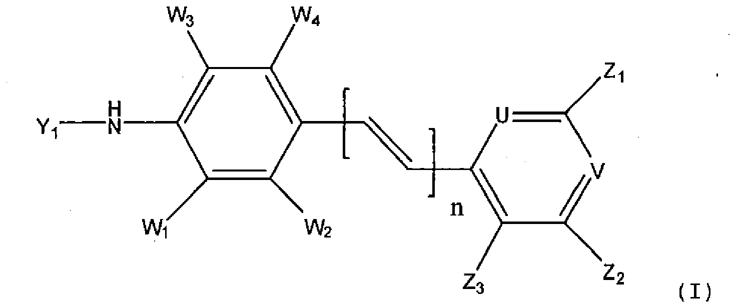 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U und V für N, N⁺R oder CZ₄ stehen, R für H, Alkyl, Aralkyl, Aryl, Alkanoyl oder Alkylsulfonyl steht
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze
b) Beimpfen des Mediums mit einer zu testenden biologischen Probe,
c) Inkubieren und
d) Bestimmen der Anwesenheit von mindestens einer Peptidase-Aktivität oder einer Veränderung des pH-Werts.

10. Medium zum Nachweis und/oder zur Identifikation von Mikroorganismen, umfassend eine Verbindung der folgenden Formel I zum Nachweisen einer Peptidase-Aktivität und/oder einer Veränderung des pH-Werts: 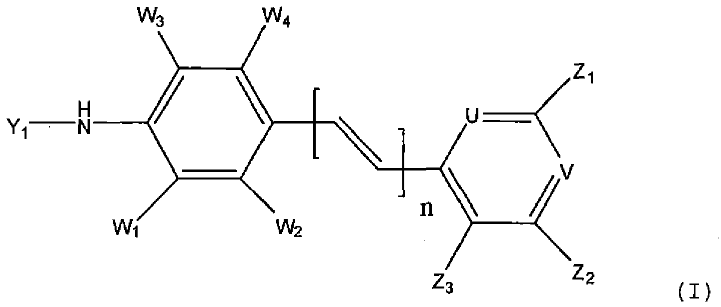 worin:
• Y₁ ein Peptid, H oder ein Alkyl ist
• W₁, W₂, W₃ und W₄ unabhängig H, Br, Cl, F, I, Alkyl, Alkoxy, Thiomethyl, Perfluoralkyl, Nitro, Cyano, Carboxyl (einschließlich dessen Ester oder Amide) oder jede Kombination von diesen sind
• n = 0, 1 oder 2
• U und V für N, N⁺R oder CZ₄ stehen, R für H, Alkyl, Aralkyl, Aryl, Alkanoyl oder Alkylsulfonyl steht
• Z₁, Z₂, Z₃ und Z₄ sind unabhängig H, Br, Cl, F, I, Alkyl, Aryl, Alkoxy, Perfluoralkyl, Nitro, Cyano, Carboxyl, Sulfonyl, einschließlich der Ester oder Amide von Carboxyl oder Sulfonyl,
und deren Salze.

## Claims

1. A use of a compound of the following formula (I), as an enzyme substrate for the detection of a peptidase activity and/or a variation in pH: 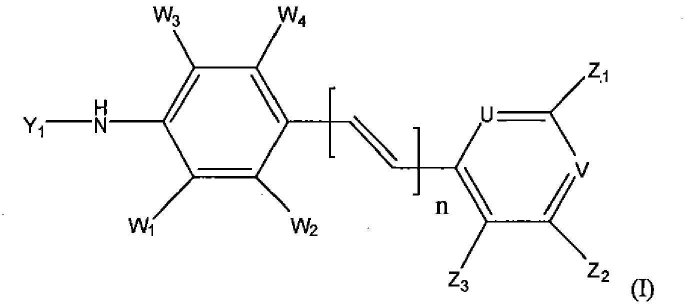 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n=0, 1 or 2;
• U and V are N, N⁺R or CZ₄, R being H, alkyl, aralkyl, aryl, alkanoyl or alkylsulfonyl;
• Z₁, Z₂, Z₃ and Z₄ being independently H, Br, Cl, F, I, alkyl, aryl, alkoxy, perfluoroalkyl, nitro, cyano, carboxyl, sulfonyl, including the carboxyl or sulfonyl esters or amides,
and salts thereof.

2. The use of a compound of formula I as claimed in claim 1, wherein Y₁ is a peptide, preferentially chosen from alanine and glycine.

3. The use of a compound of formula I as claimed in claim 1 or 2, wherein n = 1.

4. The use of a compound of formula I as claimed in any one of claims 1 to 3, wherein W₁, W₂, W₃ and W₄ are independently H.

5. The use of a compound of formula I as claimed in any one of claims 1 to 4, wherein U is CZ₄, preferentially CH.

6. The use of a compound of formula I as claimed in any one of claims 1 to 5, wherein V is N⁺R, preferentially N⁺CH₃.

7. The use of a compound of formula I as claimed in any one of claims 1 to 6, wherein Z₁, Z₂, Z₃ and Z₄ are H.

8. The use of a compound of formula I as claimed in claim 1, wherein said compound is chosen from glycyl-4-(4'-amidostyryl)-N-methylpyridinium chloride, L-alanyl-4-(4'-amidostyryl)-N-methylpyridinium TFA, β-alanyl-4-(4'-amidostyryl)-N-methylpyridinium chloride and L-alanyl-4-(4'-amidostyryl)pyridine perchlorate (unquaternized).

9. A method for the detection in microorganisms of a peptidase activity and/or of a variation in pH, comprising the following steps:
a) providing a detecting and/or identifying medium comprising a compound of the following formula (I): 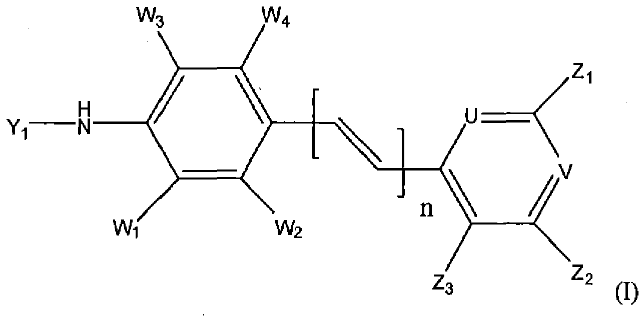 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n = 0, 1 or 2;
• U and V are N, N⁺R or CZ₄, R being H, alkyl, aralkyl, aryl, alkanoyl or alkylsulfonyl;
• Z₁, Z₂, Z₃ and Z₄ being independently H, Br, Cl, F, I, alkyl, aryl, alkoxy, perfluoroalkyl, nitro, cyano, carboxyl, sulfonyl, including the carboxyl or sulfonyl esters or amides,
and salts thereof,
b) inoculating the medium with a biological sample to be tested,
c) leaving to incubate, and
d) revealing the presence of at least one peptidase activity or a variation in pH.

10. A medium for detecting and/or identifying microorganisms comprising a compound of the following formula (I) for detecting a peptidase activity and/or a variation in pH: 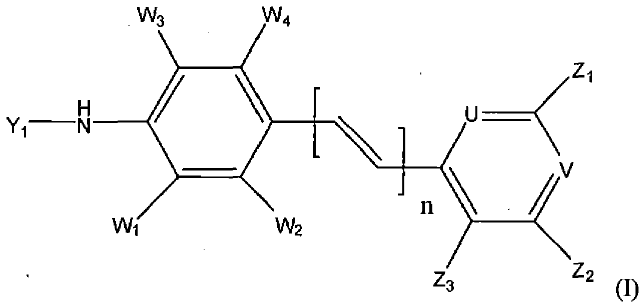 wherein:
• Y₁ is a peptide, H or an alkyl;
• W₁, W₂, W₃ and W₄ are independently H, Br, Cl, F, I, alkyl, alkoxy, thiomethyl, perfluoroalkyl, nitro, cyano, carboxyl (including the esters or amides thereof) or any combination thereof;
• n = 0, 1 or 2;
• U and V are N, N⁺R or CZ₄, R being H, alkyl, aralkyl, aryl, alkanoyl or alkylsulfonyl;
• Z₁, Z₂, Z₃ and Z₄ being independently H, Br, Cl, F, I, alkyl, aryl, alkoxy, perfluoroalkyl, nitro; cyano, carboxyl, sulfonyl, including the carboxyl or sulfonyl esters or amides,
and salts thereof.
